# EUROPÄISCHE PATENTSCHRIFT

(11) **EP 0 904 798 B1**
(45) Veröffentlichungstag und Bekanntmachung des Hinweises auf die Patenterteilung: **06.11.2002**
(21) Anmeldenummer: 97202957.3
(22) Anmeldetag: 26.09.1997
(51) Int. Cl.: A61M 29/02, A61N 5/10, A61M 25/10

(54) **Mit Kohlendioxid aufgeblasener Ballonkatheter zur Radiotherapie**
Carbon dioxide inflated radio-therapy balloon catheter
Cathéter à ballonnet pour effectuer une thérapie radiologique gonflé avec du dioxide de carbone

(43) Veröffentlichungstag der Anmeldung: 31.03.1999
(73) Patentinhaber: Schneider (Europe) GmbH, 8180 Bülach (CH)
(72) Erfinder: Popowski, Youri G., Dr., 1203 Genf (CH); Verin, Vitali E., Dr., 1202 Genf (CH)
(74) Vertreter: Kiliaridis, Constantin

(56) Entgegenhaltungen:
- EP-A- 0 360 582
- EP-A- 0 633 041
- US-A- 5 643 171

## Beschreibung

### Beschreibung für folgende Vertragsstaaten : AT, BE, CH, LI, DK, ES, IT, SE

Die Erfindung bezieht sich auf einen vorbereiteten Katheter zur intraluminalen Behandlung eines Gefässabschnittes mit ionisierender Strahlung, der einen länglichen Schaft mit einem proximalen und einem distalen Ende als perkutaner, transluminaler Zugang zum Gefässabschnitt, einen inflatierbaren Ballon am distalen Ende des Schaftes, ein sich im Schaft erstreckendes und in den Ballon mündendes Inflationslumen und eine im Ballon positionierbare Quelle ionisierender Strahlung aufweist und der mit einem Inflationsmedium zur Beaufschlagung des Ballons mit Druck gefüllt ist.

Katheter der eingangs genannten Art werden zum Beispiel bei oder nach einer perkutanen, transluminalen Angioplastie, wie etwa der Ballondilatation oder der Artherektomie eines verengten Blutgefässabschnitts, eingesetzt, um einer Restenosierung dieses Abschnitts vorzubeugen. Man geht hier davon aus, dass die Applikation einer bestimmten Strahlendosis eine durch die Angioplastie ausgelöste übermässige Zellvermehrung eindämmen kann und dass dadurch eine Wiederverengung des behandelten Gefässabschnittes vermieden werden kann. Ein gattungsgemässer Katheter kann jedoch auch für die Strahlenbehandlung anderer Körperhohlräume, wie zum Beispiel der Speiseoder Luftröhre oder zur Behandlung der Prostata, angewendet werden.

Ein Katheter der eingangs genannten Art ist aus der EP 0 633 041 A1 bekannt, bei dem in einem zentralen Führungsdrahtlumen eines zweilumigen Ballonkatheters ein Führungsdraht längsverschiebbar angeordnet ist. In die Spitze des Führungsdrahtes ist ein Emitter radioaktiver Strahlung in Form eines Filamentes integriert. Das zweite Lumen dient als Inflationslumen für den Ballon, durch dessen Inflation der im Führungdsdrahtlumen positionierte Strahlenemitter im zu behandelnden Gefässabschnitt radial zentriert wird. Dadurch wird eine über den Umfang der Gefässwand gleichmässige Strahlendosisverteilung erreicht. Zur Beaufschlagung des Ballons mit Druck wird eine für Ballondilatationen übliche physiologische Lösung verwendet. Als Strahlenquelle wird vorzugsweise Yttrium 90 eingesetzt, ein leicht abschirmbarer Betaemitter mit einer Halbwertszeit von 2,7 Tagen, einer mittleren Elektronenenergie von 0,942 MeV und einer maximalen Elektronenenergie von 2,28 MeV.

Den grössten Teil ihres Weges von dem im Ballon positionierten Emitter zur zu behandelnden Gefässwand muss die radioaktive Strahlung Inflationsmedium durchdringen, wobei - wie in jeder Materie - Strahlungsenergie absorbiert wird. Damit hängt die an der Oberfläche der Gefässwand zur Verfügung stehende Energiedosis sowie die Eindringtiefe der Strahlung in das wandnahe Gefässgewebe von der Ausgangsaktivität der Quelle, vom Absorptionskoeffizienten des Inflationsmediums und von der Weglänge der Strahlung durch das Inflationsmedium ab. Mit Kochsalzlösung oder Kontrastmittel werden üblicherweise Inflationsmittel verwendet, welche einen nicht zu vernachlässigenden Absorptionskoeffizienten aufweisen. Dadurch leiden bekannte Katheter der eingangs genannten Art unter dem Nachteil langer Bestrahlungs- und damit Behandlungszeiten. Durch die notwendige Zentrierung des Emitters in einem inflatierten Ballon muss während dieser Behandlung der Blutfluss im behandelten Gefäss unterbrochen werden. Eine hohe Absorptionsrate stellt auch hohe Anforderungen an die radiale Zentrierung der Quelle zur Erzielung einer gleichmässigen Strahlendosisverteilung.

Der Erfindung liegt daher die Aufgabe zugrunde, einen Katheter der eingangs genannten Art anzugeben, bei der ein möglichst geringer Anteil der ionisierenden Strahlung auf ihrem Weg von der Quelle durch den inflatierten Ballon zum zu behandelnden Gefässabschnitt absorbiert wird.

Die Aufgabe wird gemäss der Erfindung gelöst durch einen Katheter der eingangs genannten Art mit den Merkmalen des kennzeichnenden Teils des Patentanspruchs 1. Wenn das Inflationsmedium ein Gas ist, durchläuft die Strahlung ein Medium mit vergleichsweise geringem Absorptionskoeffizienten, da dieser im allgemeinen für Flüssigkeiten grösser ist als für Gase. Dadurch klingt die Strahlung beim Durchtritt durch das Inflationsmedium nur wenig ab, so dass dem Gefässabschnitt in kurzer Zeit eine ausreichende Strahlendosis zugeführt werden kann. Ausserdem ist die Abhängigkeit der Strahlungsintensität vom Quellenabstand schwächer, wodurch Ungenauigkeiten in der Quellenzentrierung nur geringe Auswirkungen auf die Gleichmässigkeit der Dosisverteilung haben. Darüber hinaus haben mit Gas gefüllte Ballons bis zu etwa dreimal kürzere Deflationszeiten im Vergleich zu Ballons, die mit Flüssigkeit inflatiert wurden, was auf die geringere Viskosität von Gasen im Vergleich zu Flüssigkeiten zurückzuführen ist. Dies hat den Vorteil, dass beispielsweise bei der Behandlung mit einem den Blutfluss unterbrechenden Ballon schnell auf eine ischämische Reaktion des Patienten durch Ballondeflation reagiert werden kann.

In einer bevorzugten Ausführungsform der Erfindung ist das Inflationsmedium Kohlendioxid. Bei der Behandlung von Blutgefässen kann im Falle eines undichten oder fehlerhaften Ballons das Blut eine gewisse Menge von Kohlendioxid absorbieren, ohne dem Patienten zu schaden. Da Kohlendioxid ohnehin im Blut transportiert wird, steht auch seine biologische Verträglichkeit im Menschen nicht in Frage.

In einer weiteren vorteilhaften Ausgestaltung der Erfindung weist das Inflationslumen über den grössten Teil seiner während einer Behandlung im Körper eines Patienten liegenden Länge eine Querschnittsfläche von höchstens 0,300 mm², beziehungsweise höchstens 0,200 mm² auf. Die Querschnittsfläche des Inflationslumens kann auch abhängig vom Ballonvolumen definiert werden, z.B. als in mm² ausgedrückt nicht grösser als ein maximales Gebrauchsvolumen des Ballons in mm³ dividiert durch 1 200, bzw. 1 600. Durch eine Verminderung der Querschnittsfläche des Inflationslumens kann das Gesamtprofil des Katheters verkleinert werden, wodurch ein erfindungsgemässer Katheter für minimal invasive, perkutane, transluminale Behandlungen durch kleine Punktionsöffnungen und Führungskatheter geeignet ist. Ausserdem kann der Katheter auch in entsprechend engeren Gefässen eingesetzt werden, wobei die Ballonentleerzeit immer noch klein bleibt im Vergleich zu bekannten Kathetern mit flüssigen Inflationsmedien.

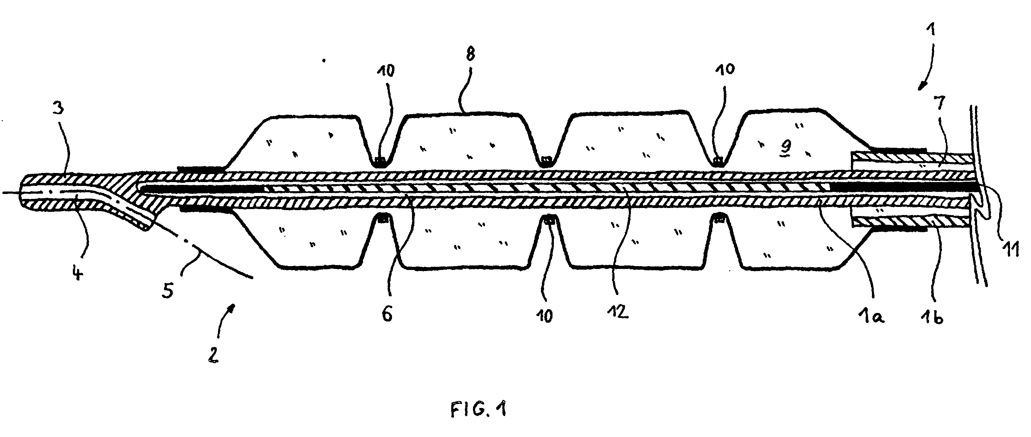

Weitere Vorteile eines erfindungsgemässen Katheters ergeben sich aus einem bevorzugten Ausführungsbeispiel, das im folgenden anhand der Zeichnung näher beschrieben wird, in deren
- FIG. 1: der distale Abschnitt eines erfindungsgemässen Katheters im Längsschnitt
veranschaulicht ist.

Gemäss FIG. 1 weist ein erfindungsgemässer Katheter zur intraluminalen Behandlung eines Gefässabschnittes mit ionisierender Strahlung einen dreilumigen Schaft 1 auf, der ein proximales (nicht dargestellt) und ein distales Ende 2 aufweist und als transluminaler Zugang zum Gefässabschnitt dient. Der Schaft 1 ist aus einem Aussenschaft 1a und einem sich in diesem koaxial erstreckenden und distal über diesen hinausragenden Innenschaft 1b aufgebaut. In einer Spitze 3 des Innenschafts 1b ist ein kurzes Führungsdrahtlumen 4 zur Aufnahme eines Führungsdrahtes (nicht dargestellt, Verlauf durch Strichpunktlinie 5 angedeutet) ausgebildet, auf welchen der Katheter zum Voranschieben im Gefässsystem aufgefädelt ist. Ein distal geschlossenes Zentrallumen 6 dient während des Einführens des Katheters zur Aufnahme eines Versteifungsdrahtes (nicht dargestellt), der beim Voranschieben des Schafts 1 axialen Schub auf die Spitze 3 überträgt. Ein zwischen Innenschaft 1a und Aussenschaft 1b verlaufendes, ringförmiges Inflationslumen 7 mündet in einen am distalen Ende 2 des Schafts 1 angeordneten Ballon 8, der über das Inflationslumen 7 mit einem Inflationsmedium 9, beispielsweise Kohlendioxid, gefüllt und dadurch inflatiert wird. Bei annähernd gleicher Entleerungszeit für den Balllon 8 ermöglicht die Verwendung von Kohlendioxid als Inflationsmedium eine Verkleinerung der Querschnittsfläche des Inflationslumens 7 z. B. auf Werte kleiner als 0,300 mm², es sind aber auch schon Querschnittsflächen kleiner als 0,200mm² hergestellt worden. Das erreichbare Verhältnis zwischen dem maximalen Gebrauchsvolumen des Ballons und der Querschnittsfläche des Inflationlumens bei tolerierbaren Entleerzeiten liegt bei ca 1 200 :1. Um einen dünnen Aussenschaft 1b zu erreichen, wurden teilweise auch die Entleerungszeiten akzeptiert, die sich aus einem Verhältnis 1 600 : 1 ergeben. Der inflatierte Ballon 8 ist von durch Ringelemente 10 gebildete Einschnürungen in mehrere Ballonsegmente unterteilt, wodurch das Zentrallumen 6 auch bei einer Verformung des Innenschaftes 1b radial zentriert ist. Ist der Ballonkatheter positioniert, so wird der Versteifungsdraht aus dem Zentrallumen 6 entfernt und durch einen Quellendraht 11 ersetzt, in den distal eine Quelle 12 ionisierender Strahlung integriert ist. Die Quelle 12 ist beispielsweise ein Filament aus Yttrium 90, welche derart im Ballon positioniert ist, dass die emittierte Betastrahlung auf ihrem Weg zur Gefässwand im wesentlichen das Inflationsmedium 9 durchdringt.

Das als Inflationsmedium verwendete Kohlendioxid steht beispielsweise in Gasflaschen unter einem Druck von z.B. 11 bar zur Verfügung. Der Gasdruck kann über ein Reduzierventil vermindert werden, sodass auch im sterilen Bereich eines Katheterlabors eine Inflationsspritze mit Kohlendioxid gefüllt werden kann. Ballon und Inflationslumen des Katheters werden wie üblich, etwa mit einer Vakuum ziehenden Spritze, entlüftet. Danach kann der Ballon mit dem von der Inflationsspritze aufgenommenen Kohlendioxid inflatiert werden.

### Bezugszeichenliste

- 1: Schaft
- 1a: Innenschaft
- 1b: Aussenschaft
- 2: distales Ende
- 3: Spitze
- 4: Führungsdrahtlumen
- 5: Führungsdrahtverlauf
- 6: Zentrallumen
- 7: Inflationslumen
- 8: Ballon
- 9: Inflationsmedium, Kohlendioxid
- 10: Ringelement
- 11: Quellendraht
- 12: Quelle

### Beschreibung für folgende Vertragsstaaten : DE, FR, GB, IE, NL

Die Erfindung bezieht sich auf einen vorbereiteten Katheter zur intraluminalen Behandlung eines Gefässabschnittes mit ionisierender Strahlung, der einen länglichen Schaft mit einem proximalen und einem distalen Ende als perkutaner, transluminaler Zugang zum Gefässabschnitt, einen inflatierbaren Ballon am distalen Ende des Schaftes, ein sich im Schaft erstreckendes und in den Ballon mündendes Inflationslumen und eine im Ballon positionierbare Quelle ionisierender Strahlung aufweist und der mit einem Inflationsmedium zur Beaufschlagung des Ballons mit Druck gefüllt ist.

Katheter der eingangs genannten Art werden zum Beispiel bei oder nach einer perkutanen, transluminalen Angioplastie, wie etwa der Ballondilatation oder der Artherektomie eines verengten Blutgefässabschnitts, eingesetzt, um einer Restenosierung dieses Abschnitts vorzubeugen. Man geht hier davon aus, dass die Applikation einer bestimmten Strahlendosis eine durch die Angioplastie ausgelöste übermässige Zellvermehrung eindämmen kann und dass dadurch eine Wiederverengung des behandelten Gefässabschnittes vermieden werden kann. Ein gattungsgemässer Katheter kann jedoch auch für die Strahlenbehandlung anderer Körperhohlräume, wie zum Beispiel der Speise- oder Luftröhre oder zur Behandlung der Prostata, angewendet werden.

Ein Katheter der eingangs genannten Art ist aus der EP 0 633 041 A1 bekannt, bei dem in einem zentralen Führungsdrahtlumen eines zweilumigen Ballonkatheters ein Führungsdraht längsverschiebbar angeordnet ist. In die Spitze des Führungsdrahtes ist ein Emitter radioaktiver Strahlung in Form eines Filamentes integriert. Das zweite Lumen dient als Inflationslumen für den Ballon, durch dessen Inflation der im Führungdsdrahtlumen positionierte Strahlenemitter im zu behandelnden Gefässabschnitt radial zentriert wird. Dadurch wird eine über den Umfang der Gefässwand gleichmässige Strahlendosisverteilung erreicht. Zur Beaufschlagung des Ballons mit Druck wird eine für Ballondilatationen übliche physiologische Lösung verwendet. Als Strahlenquelle wird vorzugsweise Yttrium 90 eingesetzt, ein leicht abschirmbarer Betaemitter mit einer Halbwertszeit von 2,7 Tagen, einer mittleren Elektronenenergie von 0,942 MeV und einer maximalen Elektronenenergie von 2,28 MeV.

Den grössten Teil ihres Weges von dem im Ballon positionierten Emitter zur zu behandelnden Gefässwand muss die radioaktive Strahlung Inflationsmedium durchdringen, wobei - wie in jeder Materie - Strahlungsenergie absorbiert wird. Damit hängt die an der Oberfläche der Gefässwand zur Verfügung stehende Energiedosis sowie die Eindringtiefe der Strahlung in das wandnahe Gefässgewebe von der Ausgangsaktivität der Quelle, vom Absorptionskoeffizienten des Inflationsmediums und von der Weglänge der Strahlung durch das Inflationsmedium ab. Mit Kochsalzlösung oder Kontrastmittel werden üblicherweise Inflationsmittel verwendet, welche einen nicht zu vernachlässigenden Absorptionskoeffizienten aufweisen. Dadurch leiden bekannte Katheter der eingangs genannten Art unter dem Nachteil langer Bestrahlungs- und damit Behandlungszeiten. Durch die notwendige Zentrierung des Emitters in einem inflatierten Ballon muss während dieser Behandlung der Blutfluss im behandelten Gefäss unterbrochen werden. Eine hohe Absorptionsrate stellt auch hohe Anforderungen an die radiale Zentrierung der Quelle zur Erzielung einer gleichmässigen Strahlendosisverteilung.

Der Erfindung liegt daher die Aufgabe zugrunde, einen Katheter der eingangs genannten Art anzugeben, bei der ein möglichst geringer Anteil der ionisierenden Strahlung auf ihrem Weg von der Quelle durch den inflatierten Ballon zum zu behandelnden Gefässabschnitt absorbiert wird.

Die Aufgabe wird gemäss der Erfindung gelöst durch einen Katheter der eingangs genannten Art mit den Merkmalen des kennzeichnenden Teils des Patentanspruchs 1. Wenn das Inflationsmedium ein Gas ist, durchläuft die Strahlung ein Medium mit vergleichsweise geringem Absorptionskoeffizienten, da dieser im allgemeinen für Flüssigkeiten grösser ist als für Gase. Dadurch klingt die Strahlung beim Durchtritt durch das Inflationsmedium nur wenig ab, so dass dem Gefässabschnitt in kurzer Zeit eine ausreichende Strahlendosis zugeführt werden kann. Ausserdem ist die Abhängigkeit der Strahlungsintensität vom Quellenabstand schwächer, wodurch Ungenauigkeiten in der Quellenzentrierung nur geringe Auswirkungen auf die Gleichmässigkeit der Dosisverteilung haben. Darüber hinaus haben mit Gas gefüllte Ballons bis zu etwa dreimal kürzere Deflationszeiten im Vergleich zu Ballons, die mit Flüssigkeit inflatiert wurden, was auf die geringere Viskosität von Gasen im Vergleich zu Flüssigkeiten zurückzuführen ist. Dies hat den Vorteil, dass beispielsweise bei der Behandlung mit einem den Blutfluss unterbrechenden Ballon schnell auf eine ischämische Reaktion des Patienten durch Ballondeflation reagiert werden kann.

In einer bevorzugten Ausführungsform der Erfindung ist das Inflationsmedium Kohlendioxid. Bei der Behandlung von Blutgefässen kann im Falle eines undichten oder fehlerhaften Ballons das Blut eine gewisse Menge von Kohlendioxid absorbieren, ohne dem Patienten zu schaden. Da Kohlendioxid ohnehin im Blut transportiert wird, steht auch seine biologische Verträglichkeit im Menschen nicht in Frage.

In einer weiteren vorteilhaften Ausgestaltung der Erfindung weist das Inflationslumen über den grössten Teil seiner während einer Behandlung im Körper eines Patienten liegenden Länge eine Querschnittsfläche von höchstens 0,300 mm², beziehungsweise höchstens 0,200 mm² auf. Die Querschnittsfläche des Inflationslumens kann auch abhängig vom Ballonvolumen definiert werden, z.B. als in mm² ausgedrückt nicht grösser als ein maximales Gebrauchsvolumen des Ballons in mm³ dividiert durch 1 200, bzw. 1 600. Durch eine Verminderung der Querschnittsfläche des Inflationslumens kann das Gesamtprofil des Katheters verkleinert werden, wodurch ein erfindungsgemässer Katheter für minimal invasive, perkutane, transluminale Behandlungen durch kleine Punktionsöffnungen und Führungskatheter geeignet ist. Ausserdem kann der Katheter auch in entsprechend engeren Gefässen eingesetzt werden, wobei die Ballonentleerzeit immer noch klein bleibt im Vergleich zu bekannten Kathetern mit flüssigen Inflationsmedien.

Weitere Vorteile eines erfindungsgemässen Katheters ergeben sich aus einem bevorzugten Ausführungsbeispiel, das im folgenden anhand der Zeichnung näher beschrieben wird, in deren
- FIG. 1: der distale Abschnitt eines erfindungsgemässen Katheters im Längsschnitt
veranschaulicht ist.

Gemäss FIG. 1 weist ein erfindungsgemässer Katheter zur intraluminalen Behandlung eines Gefässabschnittes mit ionisierender Strahlung einen dreilumigen Schaft 1 auf, der ein proximales (nicht dargestellt) und ein distales Ende 2 aufweist und als transluminaler Zugang zum Gefässabschnitt dient. Der Schaft 1 ist aus einem Aussenschaft 1a und einem sich in diesem koaxial erstreckenden und distal über diesen hinausragenden Innenschaft 1b aufgebaut. In einer Spitze 3 des Innenschafts 1b ist ein kurzes Führungsdrahtlumen 4 zur Aufnahme eines Führungsdrahtes (nicht dargestellt, Verlauf durch Strichpunktlinie 5 angedeutet) ausgebildet, auf welchen der Katheter zum Voranschieben im Gefässsystem aufgefädelt ist. Ein distal geschlossenes Zentrallumen 6 dient während des Einführens des Katheters zur Aufnahme eines Versteifungsdrahtes (nicht dargestellt), der beim Voranschieben des Schafts 1 axialen Schub auf die Spitze 3 überträgt. Ein zwischen Innenschaft 1a und Aussenschaft 1b verlaufendes, ringförmiges Inflationslumen 7 mündet in einen am distalen Ende 2 des Schafts 1 angeordneten Ballon 8, der über das Inflationslumen 7 mit einem Inflationsmedium 9, beispielsweise Kohlendioxid, gefüllt und dadurch inflatiert wird. Bei annähernd gleicher Entleerungszeit für den Balllon 8 ermöglicht die Verwendung von Kohlendioxid als Inflationsmedium eine Verkleinerung der Querschnittsfläche des Inflationslumens 7 z. B. auf Werte kleiner als 0,300 mm², es sind aber auch schon Querschnittsflächen kleiner als 0,200mm² hergestellt worden. Das erreichbare Verhältnis zwischen dem maximalen Gebrauchsvolumen des Ballons und der Querschnittsfläche des Inflationlumens bei tolerierbaren Entleerzeiten liegt bei ca 1 200 :1. Um einen dünnen Aussenschaft 1b zu erreichen, wurden teilweise auch die Entleerungszeiten akzeptiert, die sich aus einem Verhältnis 1 600 : 1 ergeben. Der inflatierte Ballon 8 ist von durch Ringelemente 10 gebildete Einschnürungen in mehrere Ballonsegmente unterteilt, wodurch das Zentrallumen 6 auch bei einer Verformung des Innenschaftes 1b radial zentriert ist. Ist der Ballonkatheter positioniert, so wird der Versteifungsdraht aus dem Zentrallumen 6 entfernt und durch einen Quellendraht 11 ersetzt, in den distal eine Quelle 12 ionisierender Strahlung integriert ist. Die Quelle 12 ist beispielsweise ein Filament aus Yttrium 90, welche derart im Ballon positioniert ist, dass die emittierte Betastrahlung auf ihrem Weg zur Gefässwand im wesentlichen das Inflationsmedium 9 durchdringt.

Das als Inflationsmedium verwendete Kohlendioxid steht beispielsweise in Gasflaschen unter einem Druck von z.B. 11 bar zur Verfügung. Der Gasdruck kann über ein Reduzierventil vermindert werden, sodass auch im sterilen Bereich eines Katheterlabors eine Inflationsspritze mit Kohlendioxid gefüllt werden kann. Ballon und Inflationslumen des Katheters werden wie üblich, etwa mit einer Vakuum ziehenden Spritze, entlüftet. Danach kann der Ballon mit dem von der Inflationsspritze aufgenommenen Kohlendioxid inflatiert werden.

### Bezugszeichenliste

- 1: Schaft
- 1a: Innenschaft
- 1b: Aussenschaft
- 2: distales Ende
- 3: Spitze
- 4: Führungsdrahtlumen
- 5: Führungsdrahtverlauf
- 6: Zentrallumen
- 7: Inflationslumen
- 8: Ballon
- 9: Inflationsmedium, Kohlendioxid
- 10: Ringelement
- 11: Quellendraht
- 12: Quelle

## Patentansprüche (Patentansprüche für folgende(n) Vertragsstaat(en): AT, BE, CH, LI, DK, ES, IT, SE)

1. Vorbereiteter Katheter zur intraluminalen Behandlung eines Gefässabschnittes mit ionisierender Strahlung, der einen länglichen Schaft (1) mit einem proximalen und einem distalen Ende (2) als perkutaner, transluminaler Zugang zum Gefässabschnitt, einen inflatierbaren Ballon (8) am distalen Ende (2) des Schaftes (1), ein sich im Schaft (1) erstreckendes und in den Ballon (8) mündendes Inflationslumen (7) und eine im Ballon (8) positionierbare Quelle (12) ionisierender Strahlung aufweist und der mit einem Inflationsmedium (9) zur Beaufschlagung des Ballons (8) mit Druck gefüllt ist,
**dadurch gekennzeichnet, dass** das Inflationsmedium (9) ein Gas ist.

2. Vorbereiteter Katheter nach Anspruch 1,
**dadurch gekennzeichnet, dass** das Inflationsmedium (9) Kohlendioxid ist.

3. Vorbereiteter Katheter nach Anspruch 1 oder 2,
**dadurch gekennzeichnet, dass** das Inflationslumen (7) über den grössten Teil seiner während einer Behandlung im Körper des Patienten liegenden Länge eine Querschnittsfläche von höchstens 0,300mm² aufweist.

4. Vorbereiteter Katheter nach Anspruch 3,
**dadurch gekennzeichnet, dass** das Inflationslumen (7) über den grössten Teil seiner während einer Behandlung im Körper des Patienten liegenden Länge eine Querschnittsfläche von höchstens 0,200mm² aufweist.

5. Vorbereiteter Katheter nach Anspruch 1 oder 2,
**dadurch gekennzeichnet, dass** das Inflationslumen (7) über den grössten Teil der während einer Behandlung im Körper des Patienten liegenden Länge eine Querschnittsfläche aufweist von einem Wert in mm² nicht grösser als ein maximales Gebrauchsvolumen des Ballons (8) in mm³ dividiert durch 1 200.

6. Vorbereiteter Katheter nach Anspruch 5,
**dadurch gekennzeichnet, dass** das Inflationslumen (7) über den grössten Teil der während einer Behandlung im Körper des Patienten liegenden Länge eine Querschnittsfläche aufweist von einem Wert in mm² nicht grösser als ein maximales Gebrauchsvolumen des Ballons (8) in mm³ dividiert durch 1 600.

## Patentansprüche (Patentansprüche für folgende(n) Vertragsstaat(en): DE, FR, GB, IE, NL)

1. Vorbereiteter Katheter zur intraluminalen Behandlung eines Gefässabschnittes mit ionisierender Strahlung, der einen länglichen Schaft (1) mit einem proximalen und einem distalen Ende (2) als perkutaner, transluminaler Zugang zum Gefässabschnitt, einen inflatierbaren Ballon (8) am distalen Ende (2) des Schaftes (1), ein sich im Schaft (1) erstreckendes und in den Ballon (8) mündendes Inflationslumen (7) und eine im Ballon (8) positionierbare Quelle (12) ionisierender Strahlung aufweist und der mit einem Inflationsmedium (9) zur Beaufschlagung des Ballons (8) mit Druck gefüllt ist,
wobei das Inflationsmedium (9) ein Gas ist, **dadurch gekennzeichnet, dass** der Katheter einen Führungsdrahtlumen (4) mit einem proximalen Eingang aufweist, welcher sich auf der distalen Seite des Ballons (8) befindet.

2. Vorbereiteter Katheter nach Anspruch 1,
**dadurch gekennzeichnet, dass** das Inflationsmedium (9) Kohlendioxid ist.

3. Vorbereiteter Katheter nach Anspruch 1 oder 2,
**dadurch gekennzeichnet, dass** das Inflationslumen (7) über den grössten Teil seiner während einer Behandlung im Körper des Patienten liegenden Länge eine Querschnittsfläche von höchstens 0,300mm² aufweist.

4. Vorbereiteter Katheter nach Anspruch 3,
**dadurch gekennzeichnet, dass** das Inflationslumen (7) über den grössten Teil seiner während einer Behandlung im Körper des Patienten liegenden Länge eine Querschnittsfläche von höchstens 0,200mm² aufweist.

5. Vorbereiteter Katheter nach Anspruch 1 oder 2,
**dadurch gekennzeichnet, dass** das Inflationslumen (7) über den grössten Teil der während einer Behandlung im Körper des Patienten liegenden Länge eine Querschnittsfläche aufweist von einem Wert in mm² nicht grösser als ein maximales Gebrauchsvolumen des Ballons (8) in mm³ dividiert durch 1 200.

6. Vorbereiteter Katheter nach Anspruch 5,
**dadurch gekennzeichnet, dass** das Inflationslumen (7) über den grössten Teil der während einer Behandlung im Körper des Patienten liegenden Länge eine Querschnittsfläche aufweist von einem Wert in mm² nicht grösser als ein maximales Gebrauchsvolumen des Ballons (8) in mm³ dividiert durch 1 600.

## Claims (Claims for the following Contracting State(s): AT, BE, CH, LI, DK, ES, IT, SE)

1. Prepared catheter for intraluminal treatment of a portion of a vessel with ionizing radiation, the catheter comprising an elongated shaft (1) with a proximal and a distal end (2) as percutaneous transluminal access to the portion of the vessel, an inflatable balloon (8) at the distal end of the shaft (1), an inflation lumen (7) extending in the shaft (1) and opening in the balloon (8) and a source of ionizing radiation (12) which can be positioned in the balloon (8) and which is filled with an inflation medium (9) to supply the balloon (8) with pressure, **characterized in that** the inflation medium (9) is a gas.

2. Prepared catheter as claimed in claim 1, **characterised in that** the inflation medium (9) is carbon dioxide.

3. Prepared catheter as claimed in claim 1 or 2, **characterised in that** the inflation lumen (7) along the major part of its length present in the body of a patient during a treatment has a maximal surface in transversal section of 0,300 mm².

4. Prepared catheter as claimed in claim 3, **characterised in that** the inflation lumen (7) along the major part of its length present in the body of a patient during a treatment has a maximal surface in transversal section of 0,200 mm².

5. Prepared catheter as claimed in claim 1 or 2, **characterised in that** the inflation lumen (7) along the major part of its length present in the body of a patient during a treatment has a surface in transversal section of a value in mm² not greater than a maximal usable volume of the balloon (8) in mm³ divided by 1 200.

6. Prepared catheter as claimed in claim 5, **characterised in that** the inflation lumen (7) along the major part of its length present in the body of a patient during a treatment has a surface in transversal section of a value in mm² not greater than a maximal usable volume of the balloon (8) in mm³ divided by 1 600.

## Claims (Claims for the following Contracting State(s): DE, FR, GB, IE, NL)

1. Prepared catheter for intraluminal treatment of a portion of a vessel with ionizing radiation, the catheter comprising an elongated shaft (1) with a proximal and a distal end (2) as percutaneous transluminal access to the portion of the vessel, an inflatable balloon (8) at the distal end of the shaft (1), an inflation lumen (7) extending in the shaft (1) and opening in the balloon (8) and a source of ionizing radiation (12) which can be positioned in the balloon (8) and which is filled with an inflation medium (9) to supply the balloon (8) with pressure, wherein the inflation medium (9) is a gas, **characterized in that** the catheter has a guidewire lumen (4) with a proximal entry situated on the distal side of the balloon (8).

2. Prepared catheter as claimed in claim 1, **characterised in that** the inflation medium (9) is carbon dioxide.

3. Prepared catheter as claimed in claim 1 or 2, **characterised in that** the inflation lumen (7) along the major part of its length present in the body of a patient during a treatment has a maximal surface in transversal section of 0,300 mm².

4. Prepared catheter as claimed in claim 3, **characterised in that** the inflation lumen (7) along the major part of its length present in the body of a patient during a treatment has a maximal surface in transversal section of 0,200 mm².

5. Prepared catheter as claimed in claim 1 or 2, **characterised in that** the inflation lumen (7) along the major part of its length present in the body of a patient during a treatment has a surface in transversal section of a value in mm² not greater than a maximal usable volume of the balloon (8) in mm³ divided by 1 200.

6. Prepared catheter as claimed in claim 5, **characterised in that** the inflation lumen (7) along the major part of its length present in the body of a patient during a treatment has a surface in transversal section of a value in mm² not greater than a maximal usable volume of the balloon (8) in mm³ divided by 1 600.

## Revendications (Revendications pour l'(les) Etat(s) contractant(s) suivant(s): AT, BE, CH, LI, DK, ES, IT, SE)

1. Cathéter préparé pour le traitement intraluminal d'une portion de vaisseau avec un rayonnement ionisant, comprenant un corps (1) allongé avec une extrémité (2) proximale et distale comme passage percutané transluminal à la portion de vaisseau, un ballonnet gonflable (8) à l'extrémité distale (2) du corps (1), une lumière de gonflage (7) s'étendant dans le corps (1) et débouchant dans le ballonnet (8) et une source (12) de radiation ionisante positionnable dans le ballonnet (8), qui est rempli avec un médium d'inflation (9) pour alimenter le ballonnet (8) en pression, **caractérisé en ce que** le médium de gonflage (9) est un gaz.

2. Cathéter préparé selon la revendication 1, **caractérisé en ce que** le médium de gonflage (9) est du gaz carbonique.

3. Cathéter préparé selon la revendication 1 ou 2, **caractérisé en ce que** la lumière de gonflage (7) sur la plus grande partie de sa longueur qui est dans le corps du patient pendant un traitement possède une surface en coupe transversale d'au maximum 0,300mm².

4. Cathéter préparé selon la revendication 3, **caractérisé en ce que** la lumière de gonflage (7) sur la plus grande partie de sa longueur qui est dans le corps du patient pendant un traitement possède une surface en coupe transversale d'au maximum 0,200mm².

5. Cathéter préparé selon la revendication 1 ou 2, **caractérisé en ce que** la lumière de gonflage (7) sur la plus grande partie de sa longueur qui est dans le corps du patient pendant un traitement possède une surface en coupe transversale d'une valeur en mm² qui n'est pas plus grande qu'un volume utile maximal du ballonnet (8) en mm³ divisé par 1 200.

6. Cathéter préparé selon la revendication 5, **caractérisé en ce que** la lumière de gonflage (7) sur la plus grande partie de sa longueur qui est dans le corps du patient pendant un traitement possède une surface en coupe transversale d'une valeur en mm² qui n'est pas plus grande qu'un volume utile maximal du ballonnet (8) en mm³ divisé par 1 600.

## Revendications (Revendications pour l'(les) Etat(s) contractant(s) suivant(s): DE, FR, GB, IE, NL)

1. Cathéter préparé pour le traitement intraluminal d'une portion de vaisseau avec un rayonnement ionisant, comprenant un corps (1) allongé avec une extrémité (2) proximale et distale comme passage percutané transluminal à la portion de vaisseau, un ballonnet gonflable (8) à l'extrémité distale (2) du corps (1), une lumière de gonflage (7) s'étendant dans le corps (1) et débouchant dans le ballonnet (8) et une source (12) de radiation ionisante positionnable dans le ballonnet (8), qui est rempli avec un médium de gonflage (9) pour alimenter le ballonnet (8) en pression, le médium de gonflage (9) étant un gaz, **caractérisé en ce que** le cathéter comprend une lumière (4) pour fil de guidage avec une entrée proximale qui se trouve sur le côté distal du ballonnet (8).

2. Cathéter préparé selon la revendication 1, **caractérisé en ce que** le médium de gonflage (9) est du gaz carbonique.

3. Cathéter préparé selon la revendication 1 ou 2, **caractérisé en ce que** la lumière de gonflage (7) sur la plus grande partie de sa longueur qui est dans le corps du patient pendant un traitement possède une surface en coupe transversale d'au maximum 0,300mm².

4. Cathéter préparé selon la revendication 3, **caractérisé en ce que** la lumière de gonflage (7) sur la plus grande partie de sa longueur qui est dans le corps du patient pendant un traitement possède une surface en coupe transversale d'au maximum 0,200mm².

5. Cathéter préparé selon la revendication 1 ou 2, **caractérisé en ce que** la lumière de gonflage (7) sur la plus grande partie de sa longueur qui est dans le corps du patient pendant un traitement possède une surface en coupe transversale d'une valeur en mm² qui n'est pas plus grande qu'un volume utile maximal du ballonnet (8) en mm³ divisé par 1 200.

6. Cathéter préparé selon la revendication 5, **caractérisé en ce que** la lumière de gonflage (7) sur la plus grande partie de sa longueur qui est dans le corps du patient pendant un traitement possède une surface en coupe transversale d'une valeur en mm² qui n'est pas plus grande qu'un volume utile maximal du ballonnet (8) en mm³ divisé par 1 600.
